# EUROPEAN PATENT APPLICATION

(11) **EP 4 799 697 A2**
(43) Date of publication of application: **02.09.2026**
(21) Application number: 26183966.6
(22) Date of filing: 09.12.2021
(51) Int. Cl.: A61P 31/12

(54) **VIRUS SPECIFIC T-CELLS FOR USE IN METHODS OF TREATING VIRAL INFECTIONS**

(30) Priority: 09.12.2020 US 202063123109 P; 08.05.2021 US 202163186098 P
(62) Document number(s) of the earlier application(s) in accordance with Art. 76 EPC: 21843818.2 / 4 259 163
(71) Applicant: Tevogen Bio Inc., Metuchen, NJ 08840 (US)
(72) Inventor: SAADI, Ryan, Metuchen, 08840 (US)
(74) Representative: V.O.

(57) **Abstract**

The invention relates to viral peptide specific cytotoxic T cells (CTLs) for use in a method for treating a viral infection; methods for producing viral peptide specific CTLs; and to pharmaceutical compositions that contain viral peptide specific CTLs.

## Description

### RELATED APPLICATIONS

The present application claims priority to U.S. Provisional Patent Application Serial Number 63/123,109, filed December 9, 2020, and U.S. Provisional Patent Application Serial Number 63/186,098, filed May 8, 2021.

### REFERENCE TO A SEQUENCE LISTING

This application contains a Sequence Listing in computer readable form. The computer readable form is incorporated herein by reference.

### BACKGROUND OF THE INVENTION

Infectious diseases were responsible for the largest global burden of premature death and disability until the end of the twentieth century, when that distinction passed to noncommunicable diseases. Of infectious disease, viral infections are a major worldwide health issue with capacity for emergent pandemic threat. For example, in the United States alone, the first case of COVID-19 was reported in January of 2020, and by August 2020, there were at least 5,821,819 identified cases and 179,708 COVID-19 attributable deaths. (Johns Hopkins Coronavirus Resource Center, 2020). While progress has been made in developing methods to treat the infection in the most seriously affected patients, virus-related mortality continues to be a significant problem, especially in specific vulnerable populations (Recovery Collaborative Group, 06/22/2020). Elderly and immune compromised individuals, those with comorbidities such as diabetes or cardiovascular disease, the unvaccinated, and minorities have higher rates of death from viral infection as compared to the remainder of the population (Guan et al., 2020; Stokes et al., 2020).

Although there are several vaccine platforms that have obtained approval for use in humans for viral infection, there remain several factors that necessitate post-infection treatment options for viruses. For example, lack of vaccination options, vaccine hesitancy, breakthrough infections in vaccinated populations, and immunological escape by viral pathogens may give rise to viral populations that no longer respond to existing anti-viral strategies such as small molecules and vaccines. Additionally, questions remain about the durability of immunity after viral infection and vaccination (To et al., 2020). There remains a need for treatment options which can be quickly adapted to address evolutional changes that occur throughout viral infection cycles, as is the case with COVID-19.

### SUMMARY OF THE INVENTION

In one embodiment, the present disclosure relates to methods of treating a viral infection. The method may comprise administering to a human patient in need thereof an effective amount of cells comprising viral peptide specific cytotoxic T lymphocytes (CTLs) that are specifically enriched cells reactive to viral peptides. The human patient may be an elderly or immunocompromised patient. The administering may be done by intravenous infusion. The infusion may be delivered to the patient through a central line or midline. The viral peptide specific CTLs may be from a single donor.

The CTLs may be sensitized against multiple peptides restricted against a single HLA allele by *in vitro* stimulation; at least 20% of the CTLs may be reactive to viral peptides; and/or the cells may comprise less than 2.5% of naïve T cells, monocytes, NK cells, or any combination thereof.

The viral peptide specific CTLs may be sensitized against one or more peptides restricted against an HLA-A1 allele, such as against one or more peptides that may be selected from Table 1. The viral peptide specific CTLs may be sensitized against one or more peptides restricted against an HLA-A2 allele, such as against one or more peptides that may be selected from Table 2 or Table 9. The viral peptide specific CTLs may be sensitized against one or more peptides restricted against an HLA-B7 allele, such as against one or more peptides that may be selected from Table 3. The viral peptide specific CTLs may be sensitized against one or more peptides restricted against an HLA-B40 allele, such as against one or more peptides that may be selected from Table 4. The viral peptide specific CTLs may be sensitized against one or more peptides restricted against an HLA-Cw7 allele, such as against one or more peptides that may be selected from Table 5. The viral peptide specific CTLs may be sensitized against a combination of viral peptides binding to any one or combination of HLA-A1, A2, B7, B40, Cw7 alleles.

The viral peptide may be from a severe acute respiratory syndrome (SARS) virus; a SARS-coronavirus 2 (COVID-19) virus; or an influenza virus.

In one embodiment, the present disclosure relates to methods of preparing viral peptide specific cytotoxic T cells (CTLs) that are specifically enriched cells reactive to viral peptides. The methods may comprise a first stimulation step, whereby a subset of monocytes may be treated to induce maturation into dendritic cells, and the dendritic cells may be pulsed with one or more virus specific peptides and co-cultured with lymphocytes for at least six days. Inducing maturation into dendritic cells may comprise a first treatment of the monocytes with GM-CSF, IL-4, or a combination of the two, for at least about 24 hours, followed by a second treatment of the monocytes with TNF-alpha, IL-1 beta, IL-6, prostaglandin E2, or any combination thereof for at least about 24 hours after the first treatment. Pulsing the dendritic cells with viral peptides may comprise incubating the dendritic cells with at least about 2 µg/mL for each viral peptide.

The methods may comprise a second stimulation step, whereby monocytes may be used to present the viral specific peptides, stimulated lymphocytes may be cultured for at least seven days, and peptide specific CTLs may be selected due to preferential adherence of T cells recognizing the pulse peptides to an adherent monocyte layer. Stimulated lymphocytes may be further selected for by treating the co-culture with human interleukin-1 (IL-1).

The methods may comprise a third stimulation step, whereby a subset of monocytes may be pulsed with multiple viral specific peptides and restricted against a single HLA allele; thereby producing viral peptide specific CTLs, wherein at least 20% of the CTLs may be reactive to viral peptides. The viral reactive CTLs may be allogeneic mononuclear leukocytes collected from a single donor.

The viral peptide specific CTLs may be sensitized against one or more peptides restricted against any one or more of an HLA-A1, A2, B7, B40, or Cw7 allele. Exemplary peptides include those set forth in Tables 1-5 and 9.

The viral peptide may be from a severe acute respiratory syndrome (SARS) virus; a SARS-coronavirus 2 (COVID-19) virus; or an influenza virus.

In one embodiment, the present disclosure relates to a pharmaceutical composition that may comprise cells comprising viral peptide specific cytotoxic T lymphocytes (CTLs) that may be specifically enriched cells reactive to viral peptides. The CTLs are from multiple donors. The CTLs may be sensitized against multiple peptides restricted against a single HLA allele by *in vitro* stimulation. At least 20% of the CTLs may be reactive to viral peptides. The cells may comprise less than 2.5% of naïve T cells, monocytes, NK cells, or any combination thereof.

The CTLs may have been sensitized against one or more viral peptides binding to one or more specific HLA-A1, A2, B7, B40, or Cw7 alleles. Exemplary peptides include those set forth in Tables 1-5 and 9.

The viral peptide specific CTLs may be specific for a virus selected from the group consisting of SARS-CoV-2 (COVID-19), influenza, parainfluenza, respiratory syncytial virus (RSV), metapneumovirus, Hepatitis B virus (HBV), Epstein-Barr virus (EBV), cytomegalovirus (CMV), BK virus (BKV), John Cunningham virus (JCV), human herpesvirus (HHV), and adenovirus. In one embodiment, the virus may be a severe acute respiratory syndrome (SARS) virus; a SARS-coronavirus 2 (COVID-19) virus; or an influenza virus.

The pharmaceutical composition may further comprise cryopreserved CTLs in DMSO, RPMl-1640, albumin, or a combination thereof; and/or one or more additional anti-viral agents.

The pharmaceutical composition may be in a form that is suitable for intravenous administration.

### BRIEF DESCRIPTION OF THE DRAWINGS

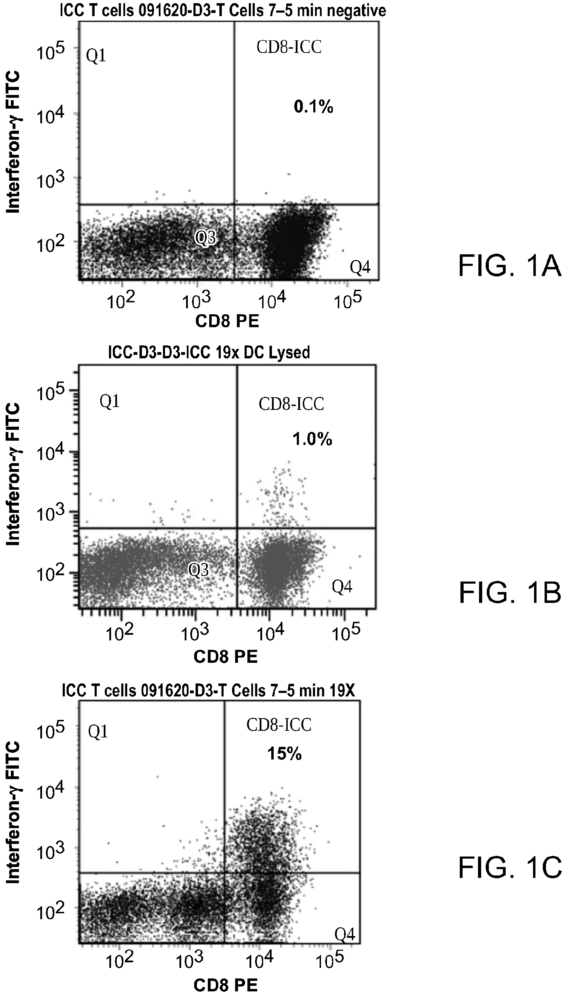

FIGs. 1A-1C are a panel of graphs showing the expansion of viral (COVID-19) CTLs through the application of proposed laboratory processes. FIG. 1A shows the background level of CD8+ CTLs, FIG. 1B shows cellular content of CD8+ CTLs after one week of *in vitro* culture, and FIG. 1C illustrates enrichment to 15% CD8+ CTLs after selection and further expansion after about three to four weeks of *in vitro* culture.

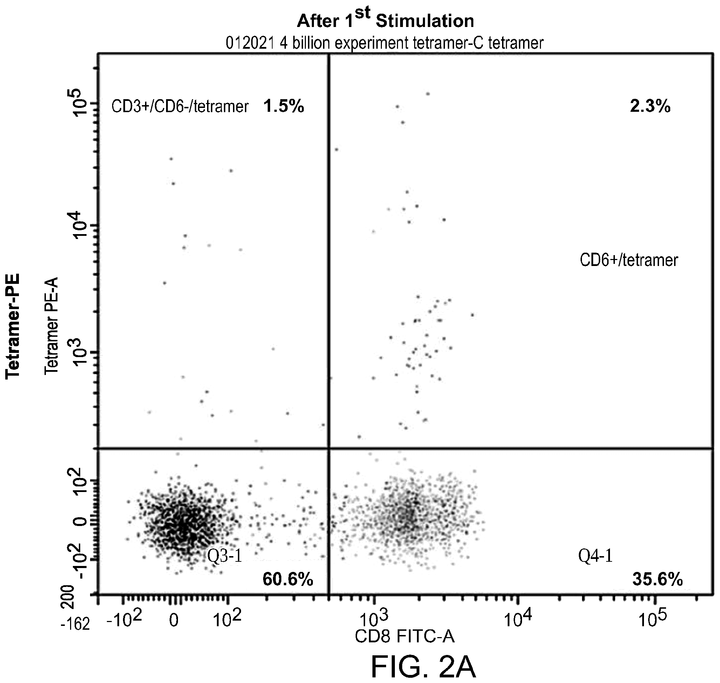

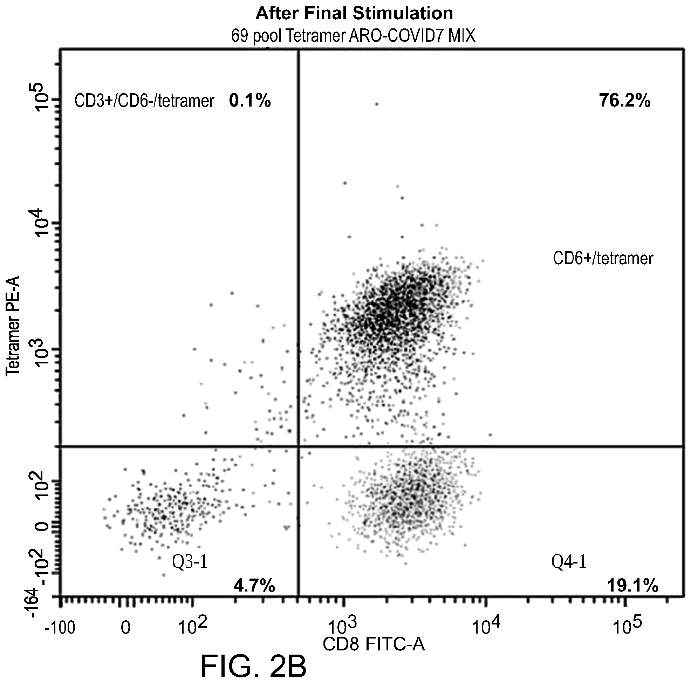

FIGs. 2A and 2B are a panel of graphs showing the results of a flow cytometry (FITC) tetramer analysis measuring CD8+ T cellular content after a first round of peptide stimulation (FIG. 2A), and after the final stimulation (FIG. 2B).

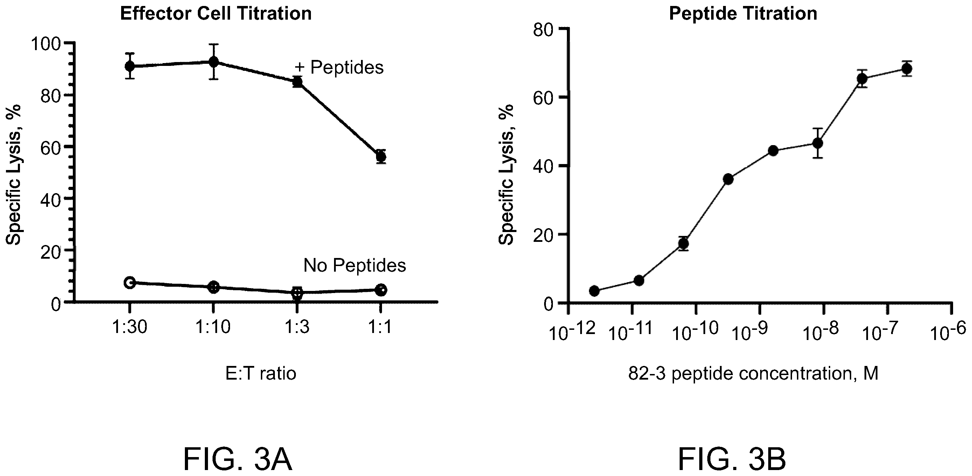

FIGs. 3A and 3B are a panel of graphs showing the results of a cytotoxicity analysis measuring virus-specific T-cell-mediated lysis as a function effector cell to T cell ratio (E:T) (FIG. 3A) and as a function of peptide concentration (FIG. 3B). In the presence of the sensitizing viral peptides, the T cells kill the targets robustly with 80% lysis at effector (cytotoxic T cell) to target (E:T) ratios as low as 3:1. Killing is at background levels in the absence of peptides.

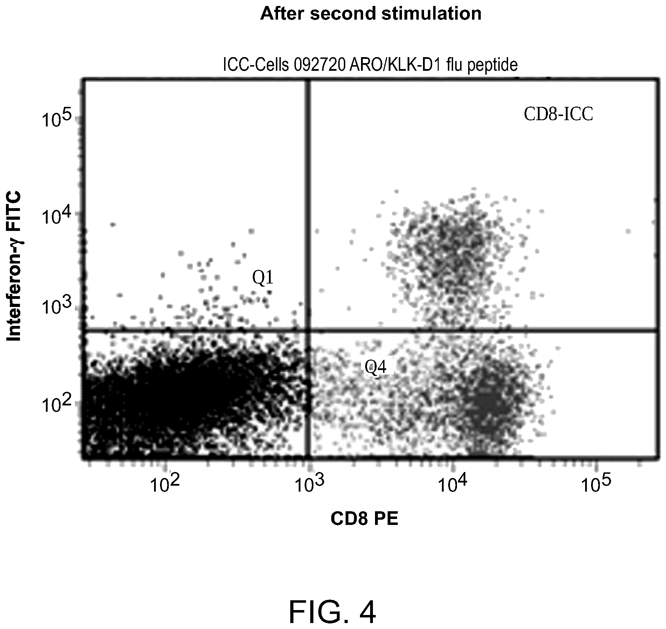

FIG. 4 is a graph showing the results of a flow cytometry (FITC) tetramer analysis measuring CD8+ T cellular content after two rounds of stimulation with influenza peptides.

### DETAILED DESCRIPTION

Applicant has developed methods for treating infected patients by administering novel preparations of viral peptide specific cytotoxic T lymphocyte (CTL) products that can be used for immunological treatment in patients who are seriously ill with a viral infection (e.g., influenza, parainfluenza, respiratory syncytial virus, metapneumovirus, Hepatitis B virus, Epstein-Barr virus (EBV), cytomegalovirus (CMV), BK virus (BKV), John Cunningham virus (JCV), human herpesvirus (HHV), adenovirus, or coronavirus. The role of T cells in eradicating viral infections provides the scientific and clinical rationale for this effort.

In addition to treating acute viral infections, virus specific CTLs may be used to prevent and/or treat viral infection associated malignancies. For example, EBV has been associated with a variety of B cell cancers including post-transplant lymphoproliferative disorder. Targeted CTLs may be used to treat other EBV associated malignancies of the B cell lineage, as well as nasopharyngeal carcinoma.

Viral infections may also set the stage for cancers down the line. Thus, there is a strong association between chronic Hepatitis B infection and the development of hepatocellular carcinoma. Eradicating the virus via targeted CTLs early after infection has the potential to avoid subsequent development of malignancy. The methods described herein are not limited to just treating the acute viral infection, but the anti-viral CTLs may be used to treat and/or prevent various types of cancers.

Existing methods of production and off-the-shelf products (e.g., AlloVir) offer a far more diffuse mixture of T cells as compared to the compositions and methods disclosed herein. Such products have been marketed for the same or similar methods disclosed herein, using terminology such as "Virus Specific T cells," or VSTs. In contrast to the commercially available products, the cells produced herein are known to be HLA class I restricted, CD8+ Cytotoxic T cells. As such, the presently disclosed methods are superior to those known in the art, as shown by the increased purity. Without wishing to be bound by theory, it is expected that the increased purity in the cells disclosed herein is due to the claimed method steps including, for example, restimulating and culturing longer during manufacture, which will eventually allow for clinical grade scalability.

At a high level, this disclosure relates to methods for treating viral infections by administering to a patient in need thereof virus specific T cells, methods of generating virus specific CTLs, and pharmaceutical compositions of virus specific CTLs. Such viral reactive CTLs may be used for diagnostic methods for detecting the presence of viruses or viral products in biological samples using virus specific T cells primed against human viruses, for example, influenza, parainfluenza, respiratory syncytial virus, metapneumovirus, Hepatitis B virus, EBV, CMV, BKV, JCV, HHV, adenovirus, and coronavirus, among other viruses. The methods described herein substantially improve upon the existing methodologies employed for generating virus peptide specific T cells by enriching for CD8+ T cells over CD4+ T cells, identifying specific HLA allele-viral peptide relationships, and result in approximately a 40-fold increase in cytotoxic efficiency of virus-specific CTLs over conventional methods.

The compositions and methods described herein can be used to focus the response on diverse targets, thereby providing a safety net that precludes the virus escaping from mutation. For example, many viral vaccines target surface-exposed glycoproteins, such as the 'S protein'. If a viral mutation develops in the S protein, then the immunological response may be compromised. Existing T cell-based therapeutic and prophylactic therapies are not rationally designed and generally include a mix of CD4 and CD8, thereby making it difficult, if not impossible, to identify the specific parts of the viral genome evoking the response. In contrast, the present invention allows one of skill in the art to identify the specific epitopes for developing immunity and with what part of the viral genome the epitopes originate. Thus, it is possible to assess the response to a variety of viral peptides separately within the overall peptide repertoire and attack the virus from a diversity of antigenic targets. As a result of the diversity included in the present invention, the virus is less likely to escape immunological detection than if the focus were placed on a single antigen, such as the S protein.

Infectious diseases are currently one of the leading causes of death worldwide, with more than 2 million deaths in 2020-2021 from coronavirus alone. Due to their relatively small genomes, rapidity of spread, progeny numbers, and strong selective pressures, among other factors, viral genomes boast very high mutation rates relative to other genetic material. Conventional viral therapies focus, for example in the case of severe acute respiratory syndrome coronavirus 2 (SARS-CoV-2), on the surface-exposed spike glycoprotein. COVID-19 variants often sport mutations in these glycoproteins, which significantly alter the folding of the Spike protein, and theoretically alter antibody binding. This may compromise the effectiveness of current therapies and prophylactic measures to new COVID-19 variants. Likewise, the duration of vaccine-induced immunity, especially to a single, mutable viral element is uncertain. Natural immunity to newly emergent viruses, such as coronaviruses, has been short-lived per some reports (Eldridge, AWD et al. Nature Medicine 2020, 26:1691-1693).

Described herein are methods of treating viral infected patients with novel viral peptide specific CTLs. Infected patients may receive an infusion of viral peptide specific CTLs manufactured using a three step (optionally, four step) process comprising *in vitro* stimulation-expansion cycles to produce the final CTL products described herein. Viral peptide specific CTLs may originate as allogeneic mononuclear leukocytes collected from one or more donors using standard leukapheresis techniques.

### Definitions

The term "pharmaceutical composition" as used herein refers to a formulation that contains an effective amount of viral peptide specific cytotoxic T lymphocytes (CTLs). A pharmaceutical composition may additionally include at least one or more pharmaceutically acceptable excipients.

The terms "administration" or "administering" as used herein refers to viral peptide specific T cells introduced to the blood of a patient. Pharmaceutical compositions of viral specific CTLs may be administered, for example, via intravenous administration to a patient.

The term "effective amount," as used herein, refers to the amount of agent needed to achieve the desired effect. The actual effective amount for a particular use can vary according to the mode of administration, and the physiological parameters of the patient, including age, weight, general health of the patient, severity of the symptoms or condition being treated, among others. Suitable amounts of viral peptide specific CTLs to be administered, and dosage schedules, for a particular patient can be determined by a clinician of ordinary skill based on these and other considerations.

The term "pharmaceutically acceptable excipient" as used herein refers to an excipient that can be administered with no significant adverse toxicological effects. Such excipients are generally regarded as safe (GRAS) by the U.S. Food and Drug Administration.

The term "viral-like illness" or "viral infection" as used herein refers to any illness or disease that presents symptoms that appear virus-like, meaning symptoms that are seen in an infection caused by a virus.

The term "CD8+ T cell" or "CD8+ cytotoxic T lymphocyte (CTL)" as used herein refers to CTLs that have CD8 co-receptors that bind to MHC class I molecules. CD8+ and CD4+ T cells have different roles: CD8+ T cells, or cytotoxic T cells, mediate killing of cells presenting non-self epitopes bound to MHC class I molecules, while CD4+ T cells regulate the immune response by recognizing a distinct set of non-self epitopes bound to MHC class II molecules. A notable difference between CD8+ and CD4+ T cells is that the efficiency of antigen presentation is better for CD8+ than for CD4+ T cells. CD8+ T cells may be detected among samples containing a heterogenous T cell population using a tetramer assay, or tetramer staining.

The term "peptide" or "viral peptide" as used herein refers to at least two contiguous amino acids covalently linked via a peptide bond. The viral peptides herein may include short amino acid sequences (on the order of ~5-20 amino acids) in length representing high-affinity ligands for a given HLA allele. The term "viral peptide" indicates that the peptide is identical to a naturally-occurring peptide sequence in a virus. The viral peptide need not be isolated from a virus, and may be generated by peptide synthesis, recombinant DNA-based peptide expression systems, or other peptide generation techniques known to the person of ordinary skill in the art. A viral peptide identical to a naturally-occurring peptide sequence in a virus may be referred to herein as being "from" that virus, even if the viral peptide is not isolated from that virus.

The term "human leukocyte antigen (HLA)" as used herein refers to human leukocyte antigen (HLA) complexes, encoded by major histocompatibility complex (MHC) genes. HLA complexes are cell-surface-displayed receptors which function to bind and display short peptides. HLA molecules are highly specific in terms of the peptide sequences they are able to present. HLA class I molecules typically bind peptides of 8-12 amino acids (aa) in length. HLA class I (HLA-I) and HLA class II (HLA-II) molecules present peptides that are typically recognized as a complex by CD8+ and CD4+ T cells, respectively.

### Methods of Treatment, Prophylaxis, and Reducing Contagion

In one aspect, the invention relates to methods for treatment and for reducing contagion of a virus (e.g., influenza, parainfluenza, respiratory syncytial virus, metapneumovirus, Hepatitis B virus, Epstein-Barr virus (EBV), cytomegalovirus (CMV), BK virus (BKV), John Cunningham virus (JCV), human herpesvirus (HHV), adenovirus, coronavirus, and the like). The methods involve administering, preferably by intravenous infusion, an effective amount of viral peptide specific T cells to a patient in need thereof. The viral peptide specific T cells may be administered by intravenous delivery to the patient, for example, by administration through a central line, midline, or peripheral IV.

Most viral infections are eradicated by T cells with a particular focus on cytotoxic T cells. The majority of therapies for viral infection have relied on measures which slow viral replication and/or moderate symptoms sufficiently to allow endogenous immunity time to develop and eradicate the virus. In contrast, viral-specific CTLs are efficacious with specific percentages of response varying with the virus, the post-transplant characteristics, persistence of immunosuppressive therapy, and other clinical variables. The CTL panels disclosed herein incorporate some of the most common HLA alleles, which provide treatment opportunities for most of the population.

In some embodiments, prior to administration of the viral peptide specific CTLs, patients will undergo full HLA typing and then be treated with an appropriate CTL.

In one embodiment, prior to administration of the viral peptide specific CTLs, patients will have their blood tested for rapid, low resolution HLA typing with high-resolution PCR sequence-specific primer (SSP) supplementation to determine if they have a potentially appropriate HLA antigen for the treatment (HLA-A1, A2, B7, B40, Cw7). The high-resolution supplementation will ensure that they are HLA-A*01:01, A*02:01, B*07:02, B*40:01, C*07:02 compatible and thus match the CTL for one or more alleles. Matching the CTL to the HLA allele(s) is necessary to match/select the correct viral antigenic peptide(s) (*see* Tables 1-5 and 14) and to ensure that the infused cells recognize viral targets likely to be present on the infected cells of the patient into whom they are introduced.

Prior to administration of the viral peptide specific CTLs, pre-medications may be administered. In some embodiments, patients may receive pre-medications, such as diphenhydramine and acetaminophen. The diphenhydramine dose may be about 15, 20, 25, or 30 mg. The acetaminophen dose may be about 500, 550, 600, 650, 700, or 750 mg.

Patients may also be treated with anti-viral drugs (e.g., remdesivir) or other standard of care pharmaceutical formulations prior to, concurrently with, or subsequently to administration of the viral peptide specific CTLs.

An effective dose of viral peptide specific CTLs is based on body weight and may be between 1 x 10⁵ total cells/kg and 3 x 10⁶ total cells/kg. A dose of 1 x 10⁵ total cells/kg, 2 x 10⁵ total cells/kg, 3 x 10⁵ total cells/kg, 4 x 10⁵ total cells/kg, 5 x 10⁵ total cells/kg, 6 x 10⁵ total cells/kg, 7 x 10⁵ total cells/kg, 8 x 10⁵ total cells/kg, 9 x 10⁵ total cells/kg, 1 x 10⁶ total cells/kg, 2 x 10⁶ total cells/kg, 3 x 10⁶ total cells/kg, 4 x 10⁶ total cells/kg, 5 x 10⁶ total cells/kg, 6 x 10⁶ total cells/kg, 7 x 10⁶ total cells/kg, 8 x 10⁶ total cells/kg, or 9 x 10⁶ total cells/kg may be administered. In some embodiments the dose will be measured by the number of virus reactive cells instead of the total amount. For example, an effective dose may be between 1 x 10⁵ virus reactive cells cells/kg and 3 x 10⁶ total cells virus reactive cells/kg. A dose of 1 x 10⁵ virus reactive cells/kg, 2 x 10⁵ virus reactive cells/kg, 3 x 10⁵ total cell virus reactive cells s/kg, 4 x 10⁵ virus reactive cells/kg, 5 x 10⁵ virus reactive cells/kg, 6 x 10⁵ virus reactive cells/kg, 7 x 10⁵ virus reactive cells/kg, 8 x 10⁵ virus reactive cells/kg, 9 x 10⁵ virus reactive cells/kg, 1 x 10⁶ total cells virus reactive cells/kg, 2 x 10⁶ virus reactive cells/kg, 3 x 10⁶ virus reactive cells/kg, 4 x 10⁶ virus reactive cells/kg, 5 x 10⁶ virus reactive cells/kg, 6 x 10⁶ virus reactive cells/kg, 7 x 10⁶ virus reactive cells/kg, 8 x 10⁶ virus reactive cells/kg, or 9 x 10⁶ virus reactive cells/kg may be administered. In some embodiments the effective dose will be based on actual body weight. In certain embodiments, where the actual weight is higher than the ideal weight, the dose will be based on adjusted body weight (ideal body weight + 40% the difference between actual and ideal weight). Ideal weight for height is calculated from the formula of BJ Devine (1974): Male: 50.0 kg + 2.3 kg per inch over 5 feet and Female: 45.5 kg + 2.3 kg per inch over 5 feet.

An "effective amount" of pharmaceutical compositions comprising viral peptide specific CTLs is administered to an individual in need thereof, such as an individual who has viral infection, has a viral-like illness, is experiencing viral-like symptoms or who is at risk for infection by a virus. An effective amount is an amount that is sufficient to achieve the desired therapeutic or prophylactic effect, such as an amount sufficient to reduce virus, viral-like illness or viral-like symptoms, to reduce duration of illness, to reduce virus titer in an individual, to reduce the number of days that infected individuals experience viral-like symptoms and/or require oxygen by any means, to reduce the number of patients who develop viral related cytokine release syndrome, and/or to decrease the incidence or rate of virus infection. A clinician of ordinary skill can determine appropriate dosage and optionally, anti-viral agent, based on, for example, the individual's age, sensitivity, tolerance and overall well-being. The viral peptide specific CTLs can be administered in a single dose or multiple doses as indicated.

Intravenous delivery of the CTLs (e.g., infusion through a peripheral line, central line or midline) should take less than 10 minutes. In some embodiments the time to infuse the CTLs is about 10 minutes, about 9 minutes, about 8 minutes, about 7 minutes, about 6 minutes, about 5 minutes, about 4 minutes, about 3 minutes, about 2 minutes, or about 1 minute.

In one embodiment, the method comprises administering an effective amount of a pharmaceutical composition to an individual suspected of having a virus, with confirmed virus or at risk for virus (e.g., at risk for infection by coronavirus, influenza, parainfluenza, respiratory syncytial virus, metapneumovirus, Hepatitis B virus, EBV, CMV, BKV, JCV, HHV, adenovirus, and the like). The methods also comprise administering an effective amount of a pharmaceutical composition to an individual with viral-like illness.

The pharmaceutical compositions may be intended for administration to the blood of a patient, and can be administered in any suitable form, such as intravenously.

In some aspects, the therapeutic method comprises administering to an individual suspected of having a virus or at risk of having a virus an effective amount of a pharmaceutical composition of the invention. For example, in some embodiments the individual is suspected of having a virus (e.g., coronavirus, influenza, parainfluenza, respiratory syncytial virus, metapneumovirus, Hepatitis B virus, EBV, CMV, BKV, JCV, HHV, adenovirus, and the like) and may have one or more symptoms of a virus. Symptoms of viruses are well-known and may include, for example, fever, cough, and shortness of breath, diarrhea, cystitis/bloody urine, hepatitis, depending on the particular virus. Additional symptoms of some viral infections may include difficulty breathing, persistent pain or pressure in the chest, confusion, inability to arouse, bluish lips or face.

In some embodiments, the method is for treating a viral infection, and comprises administering to an individual in need thereof an effective amount of a pharmaceutical composition of the invention. In other embodiments, the method is for the prophylaxis of viral infection and comprises administering to an individual at risk for infection by a virus (e.g., coronavirus, influenza, parainfluenza, respiratory syncytial virus, metapneumovirus, Hepatitis B virus, EBV, CMV, BKV, JCV, HHV, adenovirus) an effective amount of a pharmaceutical composition of the invention. In other embodiments, the method is for reducing the spread of viral infection comprising administering to an individual infected by a virus (e.g., coronavirus, influenza, parainfluenza, respiratory syncytial virus, metapneumovirus, Hepatitis B virus, EBV, CMV, BKV, JCV, HHV, adenovirus) or at risk for infection by a virus (e.g., coronavirus, influenza, parainfluenza, respiratory syncytial virus, metapneumovirus, Hepatitis B virus, EBV, CMV, BKV, JCV, HHV, adenovirus) an effective amount of a pharmaceutical composition of the invention.

Suitable intervals between doses that provide the desired therapeutic effect can be determined based on the severity of the condition (*e.g*., infection), overall well-being of the patient and the patient's tolerance to the pharmaceutical compositions, and other considerations. Based on these and other considerations, a clinician can determine appropriate intervals between doses. Generally, a pharmaceutical composition is administered once, but may be administered every one to four days, or once a week, as needed.

The therapeutic methods and uses of the invention provide particular benefits when the individual suspected of having a virus, with confirmed virus, at risk for viral infection (e.g., adults over 60 years, those with serious chronic medical conditions (such as heart disease, diabetes, lung disease), immunocompromised individuals, patients with recent cancer treatment, or with viral-like illness who also has a pulmonary disease, such as asthma (*e.g.*, allergic/atopic, childhood, late-onset, cough-variant, or chronic obstructive), airway hyperresponsiveness, allergic rhinitis (seasonal or non-seasonal), bronchiectasis, chronic bronchitis, emphysema, chronic obstructive pulmonary disease, cystic fibrosis, early life wheezing, and the like. These patient populations are particularly susceptible to viral and other respiratory infections, and these infections are frequent causes of acute exacerbation of the underlying pulmonary disease. Accordingly, the methods and therapeutic uses described herein can provide additional benefit in these patient populations by reducing the incidence, duration and/or severity of acute exacerbations of the underlying pulmonary disease.

After administration, successful treatment will be determined by testing patient blood, urine, or stool samples, and/or nasal or nasopharyngeal swab specimens for viral diagnostics, CTL persistence, the formation of endogenous CTL and antibody responses to virus. Responses to treatment may be tested, for example, at 4 days, 7 days, 14 days, 28 days, 2 months, 3 months, and 6 months post-infusion.

### Methods for Preparing Viral Peptide Specific CTLs

The viral peptide specific CTLs used in the methods of treatment provided herein can be prepared using any suitable method, for example allogeneic mononuclear leukocytes can be collected from a donor using standard leukapheresis techniques and then sensitized with viral peptides. The lymphocytes can be exposed to a limited number of peptides known to bind to the HLA restriction element of interest. For example, lymphocytes may be stimulated with 1, 2, 3, 4, 5, 6, 7, 8, 9, 10, 11, 12, 13, 14, 15, 16, 17, 18, 19, 20, 1 to 5, 1 to 10, 1 to 15, 1 to 20, 2 to 5, 2 to 10, 2 to 15, 2 to 20, 5 to 10, 5 to 15, 5 to 20, 10 to 20, or 15 to 20 peptides known to bind to the HLA restriction element of interest. The viral specific CTLs may be derived from peripheral blood lymphocytes.

For example, the HLA restriction element of interest may be selected from three classical HLA-I genes expressed in all nucleated cells in humans: HLA-A, HLA-B, and HLA-C. HLA-I molecules present peptides derived from intracellular proteins. The intracellular antigen presentation pathway may involve cleavage of viral proteins in the cytosol by proteasomes, translocation to the endoplasmic reticulum (ER) lumen, trimming by ER-resident aminopeptidases, loading onto HLA and presentation at the cell surface. HLA-II genes (HLA-DR, HLA-DP and HLA-DQ) are constitutively expressed in only a subset of cells specialized for antigen presentation, such as dendritic cells, B cells, and macrophages, but expression can also be induced in additional cell types, e.g. in response to cytokine stimulation. HLA-II molecules present peptides derived from extracellular proteins taken into cells via endocytosis and phagocytosis, and intracellular proteins that access the HLA-II processing pathway via autophagy.

HLA-I molecules typically bind peptides of 8-12 amino acids (aa) in length. The HLA-I peptide-binding cleft is closed at both N-and C-terminal ends, and optimal length preferences are often biased towards binding of ~9-mer peptides. For most HLA-I alleles the length preferences differ between alleles. High affinity ligands for a given HLA allele usually share a common amino acid motif with relatively strict preferences in anchor positions (for HLA-I usually the second (P2) and last (PΩ), for HLA-II -P1, P4, P6 and P9), which form specific interactions with residues of corresponding HLA binding pockets. The HLA locus is the most polymorphic in the human genome with tens of thousands alleles described to date. HLA variants that differ in peptide-contacting residues differ in the repertoire of peptides they present. The diversity of HLA alleles in the population is an important evolutionary mechanism for defense against diverse pathogens, e.g. rapidly mutating viruses, newly emergent viruses, and the like. HLA alleles may be associated with the severity and outcomes of viral infections. For example, the HLA-C* 15:02 allele is associated with protection against SARS-CoV-1, and HLA-B57 is highly associated with efficient HIV-1 control and long-term non-progressive infection in the absence of antiretroviral therapy.

The specific peptides used in this method are distinct for each virus and will change over time as new peptides are discovered, and existing peptides are demonstrated experimentally to elicit poor CTL outcomes. The list of peptides provided in this application is not exhaustive, and will continue to evolve as a dynamic list, but is provided as a nonlimiting exemplary list.

Peptides used in methods of generating viral peptide specific CTLs, methods of treating and preventing viral infection using viral peptide specific CTLs, and pharmaceutical compositions of viral peptide specific CTLs, as described herein, can originate from any virus. For example, T cell epitopes have been identified, collected, and reported for a wide range of viruses in the Immune Epitope Database and Analysis Resource (IEDB).

In one aspect, lymphocytes undergo three *in vitro* stimulation-expansion cycles to produce the final CTL products used in the methods of treatment described herein. Each of these three stimulation-expansion cycles has a different purpose within the overall production process, and each therefore follows a distinct procedure. Optionally, a fourth restimulation may be performed. The fourth restimulation may be performed (1) for products which fall slightly short of meeting release criteria when it is anticipated that an additional round of stimulation and expansion will allow the product to meet these criteria or (2) if additional cell expansion is desired and it is thought that an additional round of stimulation and expansion will likely significantly increase the number of treatment doses which can be obtained from that batch. Any such optional fourth restimulation may be performed following the identical process for the third stimulation.

In one embodiment, mononuclear cells from healthy volunteer donors are separated by elutriation into lymphocyte and monocyte fractions. Lymphocytes are stimulated with peptides derived from the known sequence of the viral genome and predicted/demonstrated to bind to specific HLA alleles. Viral-derived products are not utilized. In the first stimulation, a subset of the collected monocytes is treated so as to induce their maturation into dendritic cells. Dendritic cells are then pulsed with one or more viral-specific peptides and co-cultured with the lymphocytes for 7 days. The second and third stimulations utilize monocytes to present the peptides and again allow 7-12 days for stimulated lymphocytes to grow/expand. The second stimulation also includes an enrichment step which helps to select for peptide specific CTLs (due to preferential adherence of T cells recognizing the pulse peptides to an adherent monocyte layer) and reduces the content of other non-specific 'bystander' lymphocytes or other immune cells from the donor. The third stimulation again uses monocytes and peptides but does not repeat this selection step.

Most of the procedures described herein are performed in RPMI-1640 media with 10% heat-inactivated AB serum. The amount of AB serum in the media may be reduced to 9%, 8%, 7%, 6%, 5%, 4%, 3%, 2%, or 1%. Serum-free media and autologous plasma may be used as alternatives. This is referred to as "complete media" or CM. Any suitable media may be used, as determined by one of skill in the art, such as AIM V or other serum free media preparations alone, with 10 % or lower concentrations of pooled serum or autologous serum or plasma, RPMI-1640 with serum substitutes with or without lower concentrations of pooled serum or autologous serum or plasma.

In a particular embodiment, the first stimulation, referred to as the Initial *In Vitro* Sensitization, lymphocytes are stimulated with peptides for a given HLA allele as a pool, not as individual peptides. An exemplary initial list of peptides for the five HLA alleles are included in Examples 1-20 below (*see* Tables 1-5). The list contains coronavirus suitable peptides and is expected to expand as new information continues to become available, and one of ordinary skill will be able to identify additional peptides for use in the methods described herein. It is also possible that some peptides from the initial list will be removed.

Another exemplary list of peptides for the HLA-A2 allele is included in Examples 13-14, Table 9. Table 9 lists influenza-suitable peptides. We expect this list to expand as new information continues to become available. One of ordinary skill will be able to identify additional peptides for use in the methods described herein. It is also possible that some peptides listed in Table 9 may prove ineffective.

For the initial *in vitro* sensitization, dendritic cells are used as antigen presenting cells. These cells are prepared from elutriated monocytes. Fresh or freshly thawed monocytes can be enriched by adherence to plastic. A suitable number of monocytes are resuspended in media and then cells are transferred to a tissue culture plate. It is expected that approximately half of all monocytes originally used will mature into antigen-presenting dendritic cells (DCs) usable for the method. The cells are then incubated for a suitable time (e.g., at least 60 minutes, at least 90 minutes, at least 120 minutes) to allow the monocytes to adhere to the culture plate. After incubation, the supernatant is aspirated from the culture plate. Adherent cells may then then be cultured with media supplemented with granulocyte-macrophage colony-stimulating factor (GM-CSF) and IL-4 for a suitable time (e.g., 24 hours), at which point maturation cytokines (e.g., TNF-alpha, IL-1 beta, IL-6, and/or prostaglandin E2) may be added. This process is used to force the monocytes to differentiate into antigen presenting DCs. After incubation for 24 hours, the dendritic cells may detach and are ready for harvest by aspiration and centrifugation. The duration of culture in maturation cytokines may, if necessary, be extended beyond 24 hours to about 30 hours, about 36 hours, about 42 hours, or up to about 48 hours.

Following their harvest, the dendritic cells are pulsed with peptides (e.g., at a concentration of 2 microgram/mL [µg/mL] each) for a suitable length of time (e.g., for about 60, 75, 90, or 120 minutes). These dendritic cells will then present the viral peptides and be co-cultured thereafter with lymphocytes in tissue culture flasks (e.g., 75 cm²) in media (e.g., CM). The ratio of lymphocytes to dendritic cells in culture may be 15:1, 16:1, 17:1, 18:1, 19:1, 20:1, 21:1, 22:1, 23:1, 24:1, or 25:1. Lymphocytes (e.g., a total of 80 x 10⁶ cells, 100 x 10⁶ cells, or 120 x 10⁶ cells) are then added to each flask. This is considered day 0 of the CTL culture process. Cultures are not disturbed during this stimulation for 7 days to allow cell-cell interactions to form.

After approximately 7 days, the second stimulation enrichment and subsequent expansion of viral specific CTLs occurs. Seven days after the initial sensitization, CTLs are re-stimulated as part of an enrichment step which helps to select for peptide specific CTLs and reduces the content of other non-specific 'bystander' lymphocytes or other immune cells from the donor. Enrichment is based on preferential adherence of peptide specific CTLs to a monocyte layer which has been pulsed with the specific viral peptides used for the initial sensitization. CTLs recognizing any of the peptides as presented by the appropriate HLA allele will preferentially adhere to the peptide-pulsed monocyte layer through creation of an immunologic synapse in contrast to 'bystander' lymphocytes, which in the absence of such interactions can be gently washed away. While some 'bystander' lymphocytes may non-specifically adhere, this typically allows for an approximately 10-fold or greater enrichment of peptide specific CTLs versus the starting material. To perform this enrichment step, monocytes (e.g., 10 x 10⁶ cells/mL) are added to tissue culture plates. Peptides are added (e.g., at a final concentration of 2 µg/mL each) and allowed to incubate with the monocytes (e.g., for about 90 minutes). Lymphocytes (e.g., 60 x 10⁶ cells/mL, 70 x 10⁶ cells/mL, 80 x 10⁶ cells/mL, 90 x 10⁶ cells/mL, 100 x 10⁶ cells/mL, 110 x 10⁶ cells/mL, or 120 x 10⁶ cells/mL, typically the contents of one of the 75 cm² tissue culture flask) are then added to the wells. 'Bystander' lymphocytes are removed from the wells by gentle washing with PBS after an appropriate length of time (e.g., about 5, 7.5, 10, or 12 minutes). Adherent lymphocytes are allowed to remain in contact with peptide-pulsed monocytes overnight to complete the activation/restimulation process.

The following day, lymphocytes are removed from monocyte layers. The adherent lymphocytes from the prior stage are dislodged and transferred to tissue culture flasks in media with recombinant human IL-2 (e.g., at a concentration of 50 U/ml). IL-2 is added over time (e.g., 50 U/mL every 48 hours) to facilitate optimal growth of the stimulated CD8+ T cells. Media is changed if and when flasks show conversion to higher acidity (a more orange/yellow color from phenol red in the culture) via metabolic waste build-up. Media may also be changed when the glucose level drops below 60 mg/dl, or the lactate rises to over 11.5 mmol/L. Cells are then cultured for a total of 7 days following the second stimulation. Enrichment on the monocyte layer as part of this second stimulation is critical and provides a true advantage over prior CTL cultivation methods. It is believed that the enrichment on the monocyte layer is responsible for the significantly higher purity level amongst total T cells that has not been achieved prior to the present invention. This purification step may correspondingly increase selection for viral peptide specific CD8+ CTLs over CD4+ and other non-specific bystander lymphocytes, resulting in final products that contain homogenous > 90% pure samples.

A third stimulation is then performed to further expand the viral peptide specific CTLs. The enrichment step performed as part of the second stimulation is not typically repeated as part of the third stimulation. However, if assessment of the percentage of viral reactive lymphocytes (as measured by intracellular cytokine assay or tetramer assay) is below a certain limit (e.g., about 12-18%, 12%, 13%, 14%, 15%, 16%, 17% or 18%) within a day of the planned third stimulation, the procedure for the second stimulation may be repeated in lieu of the usual procedure for the third stimulation. Of the entire CTL production process, the enrichment step involves the most manipulation and is the point most vulnerable to introduction of contamination and it is thus desirable to avoid repeating this step more than once to the extent this is feasible. Further enrichment of percentage of viral peptide specific CTLs is anticipated after the third stimulation, even without repeating the enrichment step. This reflects the fact that stimulated cells will grow in IL-2 containing media where unstimulated 'bystander' cells will not. Over time, unstimulated 'bystander' cells will die off in culture leading to a more enriched virus-specific CTL product. By setting the above threshold for when the enrichment step from the second stimulation may be repeated, it is anticipated that it will be repeated infrequently, and only when essential to the manufacturing process.

After approximately seven days of enrichment culture in the tissue culture flask (e.g., day 22, 23, or 24 of CTL stimulation/culture overall), cells are counted, tested for viability, and restimulated with monocytes and peptide in cell culture flasks, such as cell culture flasks commercially available under the brand names G-Rex^{®} (Wilson Wolf Manufacturing Corporation, St. Paul, MN) or T75 (Thermo Fisher Scientific, Waltham, MA). The lymphocyte:monocyte ratio may be between 4:1 and 5:1, 4:1, 4.1:1, 4.2:1, 4.3:1, 4.4:1, 4.5:1, 4.6:1, 4.7:1, 4.8:1, 4.9:1, or 5:1. Each peptide is again added at a concentration of 2 µg/mL. Lymphocytes (1.5x10⁷ cells) are added to each cell culture flask for this secondary restimulation. Sensitization is performed in media (e.g., 40 mL of complete media) with IL-2 (e.g., 50 U/mL) supplementation.

Following the third stimulation, CTLs are again cultured (e.g., for 7 days). Media and IL-2 change may be performed every 3-4 days depending on when media color change is observed.

Following conclusion of the full three and a half week period of stimulation and expansion from day 0, CTLs will be assessed as to whether they meet the necessary criteria and, if so, harvested for cryopreservation (e.g., within 24 hours thereafter). Optionally, in some circumstances a fourth stimulation may be performed following the guidelines for the third stimulation. This will typically be performed when further cell expansion is deemed desirable to increase the number of doses of CTLs being generated or if products fall slightly short of release criteria and it is thought that an additional round of stimulation/expansion will allow the product to meet all criteria. Restimulation steps may be performed at 6-10 day intervals (e.g., 6 day, 7 day, 8 day, 9, day, 10 day intervals), though these steps may occur up to one day earlier or later than this typical 7-day interval, if necessary.

After completion of the three *in vitro* stimulation-expansion cycles products are then screened for appropriate cellular content, function, viability and sterility. Cellular content may be screened using tetramer analysis (e.g., making use of fluorescein isothiocyanate (FITC)) to determine the concentration of CD8+ and/or CD3+CD8+ CTLs in the sample and the overall heterogeneity of the sample.

Appropriate cellular content means at least 20% (e.g., at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%) of the cells will respond to viral peptides based on intracellular cytokine (ICC) staining, or tetrameter binding, and that the content of naïve T cells, monocytes, and NK cells in the product is less than 2.5% (e.g., about 2.4%, about 2.3%, about 2.2.%, about 2.1%, about 2.0%, about 1.9%, about 1.8%, about 1.7%, about 1.6%, about 1.5%, about 1.4%, about 1.3%, about 1.2%, about 1.1%, about 1.0%, about 0.9%, about 0.8%, about 0.7%, about 0.6%, about 0.5%, about 0.4%, about 0.3%, about 0.2%, or about 0.1%). Preferably, at least 70%, of the cells will respond to viral peptides based on tetrameter binding.

CTLs for use in methods of generating virus specific CTLs, methods of treating and preventing viral infection using virus specific CTLs, and pharmaceutical compositions of virus specific CTLs, as described herein, are substantially CD8+ T cells by cellular content. CTLs described herein include at least about 60% CD8+ T cells, at least about 70% CD8+ T cells, at least about 80% CD8+ T cells, at least about 85% CD8+ T cells, at least about 90% CD8+ T cells, at least about 95% CD8+ T cells, or at least about 99% CD8+ T cells by total cellular content. CTLs can begin as CD8+ enriched cellular compositions, where all other lymphocyte types have been depleted. CD8+ T cell content can be determined using flow cytometry with anti-CD8 antibodies.

Of the CD8+ T cell population making up the CTLs, the CD8+ T cells can be at least about 60% peptide specific CD8+ T cells, at least about 70% peptide specific CD8+ T cells, at least about 80% peptide specific CD8+ T cells, at least about 85% peptide specific CD8+ T cells, at least about 90% peptide specific CD8+ T cells, at least about 95% peptide specific CD8+ T cells, at least about 99% peptide specific CD8+ T cells by cellular content.

CTLs for use in methods of generating virus specific CTLs, methods of treating and preventing viral infection using virus specific CTLs, and pharmaceutical compositions of virus specific CTLs, as described herein, are substantially depleted of CD4+ T cells by cellular content. CTLs described herein include at least about 30% or less of CD4+ T cells, at least about 20% or less of CD4+ T cells, at least about 15% or less of CD4+ T cells, at least about 10% or less of CD4+ T cells, at least about 5% or less of CD4+ T cells, at least about 2.5% or less of CD4+ T cells, at least about 2.0% or less of CD4+ T cells, at least about 1.5% or less of CD4+ T cells, at least about 1.0% or less of CD4+ T cells by total cellular content.

CTLs for use in methods of generating virus specific CTLs, methods of treating and preventing viral infection using virus specific CTLs, and pharmaceutical compositions of virus specific CTLs, as described herein, are substantially depleted of naïve T cells, NK cells, monocytes, dendritic cells (DCs), and/or B cells. CTLs for use in methods of treatment herein have a total cellular content of naïve T cells, NK cells, monocytes, DCs, and/or B cells that is at least about 2.5% or less of total cellular content (e.g. about 2.4%, about 2.3%, about 2.2.%, about 2.1%, about 2.0%, about 1.9%, about 1.8%, about 1.7%, about 1.6%, about 1.5%, about 1.4%, about 1.3%, about 1.2%, about 1.1%, about 1.0%, about 0.9%, about 0.8%, about 0.7%, about 0.6%, about 0.5%, about 0.4%, about 0.3%, about 0.2%, or about 0.1%), either individually or in aggregate. These cell populations were thought to be of concern in triggering graft versus host disease (e.g. naïve T cells), HLA alloimmunization, transfer of leukotropic pathogens, or leading to immune exhaustion, among other cell therapy complications.

Function is based on at least about 40% cytolytic activity of the CTL toward peptide pulsed targets at an effector:target ratio of 40:1. In some embodiments function is based on about 80% cytolytic activity of the CTLs of peptide pulsed targets at 30:1, 10:1, and up to 3:1 effector to target ratio.

Viability should exceed 70%, for example viability of 75%, 80%, 85%, 90%, 95%, or 99% may be appropriate. Virus-specific CTLs may be amenable for cryopreservation.

The approach can be scaled to yield sufficient cells for experimentation, treatments, or clinical trials. For example, our proposed phase I-II trial required approximately 3.5 x 10⁹ CTLs (total cell count) to support the trial. The method described herein began with 2.9 x 10⁹ lymphocytes isolated from donors and resulted in 9.2 x 10⁹ lymphocytes, at least 76% of which were detectable from tetramer analysis as CD8+, enough material for clinical trials.

Sterility may be assessed through routine bacterial and fungal cultures, as well as assays for mycoplasma and endotoxin. CTLs may be cryopreserved in cryobags at any desired concentration, for example a concentration of about 2 x 10⁶ viable cells/mL, and stored for later use in the methods disclosed herein.

### Pharmaceutical Compositions Containing Viral Peptide Specific Cytotoxic T cells (CTLs)

In one aspect, the invention relates to pharmaceutical compositions for intravenous delivery that contain viral peptide specific CTLs. The pharmaceutical compositions are for intravenous delivery to an individual in need thereof, for example, by infusion through a peripheral IV, central line or midline catheter. The pharmaceutical compositions typically also include one or more carriers or excipients that are suitable for delivery of cryopreserved CTLs, such as DMSO, and the like.

In one embodiment, the pharmaceutical composition comprises viral specific CTLs that have been sensitized against SARS-CoV-2 (COVID-19) peptides binding to specific HLA-A2 alleles (e.g., SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, or a combination thereof).

In one embodiment, the pharmaceutical composition comprises viral specific CTLs that have been sensitized against COVID-19 peptides binding to specific HLA-A1 alleles (e.g., SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, or a combination thereof).

In one embodiment, the pharmaceutical composition comprises viral specific CTLs that have been sensitized against COVID-19 peptides binding to specific HLA-B7 alleles (e.g., SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, or a combination thereof).

In one embodiment, the pharmaceutical composition comprises viral specific CTLs that have been sensitized against COVID-19 peptides binding to specific HLA-B40 alleles (e.g., SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, or a combination thereof).

In one embodiment, the pharmaceutical composition comprises viral specific CTLs that have been sensitized against COVID-19 peptides binding to specific HLA-Cw7 alleles (e.g., SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, or a combination thereof).

In one embodiment, the pharmaceutical composition comprises viral specific CTLs that have been sensitized against COVID-19 peptides binding to specific HLA-Cw7 alleles (e.g., SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, or a combination thereof).

In another embodiment, the pharmaceutical composition comprises viral specific CTLs that have been sensitized against influenza virus peptides binding to specific HLA-A2 alleles (e.g., SEQ ID NOS 78-87, or a combination thereof).

In another embodiment, the pharmaceutical composition comprises viral specific CTLs that have been sensitized against one or more viral peptides binding to any one or combination of HLA-A1, A2, B7, B40, Cw7 alleles. In another embodiment, the pharmaceutical composition comprises viral specific CTLs that have been sensitized against a combination of viral peptides binding to a combination of alleles (e.g., a half dose of A2 CTL combined with a half dose of B7 CTL).

In some embodiments, the pharmaceutical composition comprises cryopreserved CTLs in DMSO, RPMI-1640, albumin, or a combination thereof.

If desired, the pharmaceutical compositions described herein can also include one or more additional anti-viral agents, such as remdesivir. Anti-viral agents suitable for intravenous injection may include acyclovir, peramivir, zanamivir, oseltamivir, ganciclovir, foscarnet, and the like, may also be included in pharmaceutical compositions of CTLs for patient use, or provided as a co-therapy.

The pharmaceutical composition can be in any form that is suitable for intravenous administration.

### Exemplification

### Example 1: Pretreatment Period/Screening

Patients with a documented diagnosis of viral infection will have their blood tested for rapid, low resolution human leukocyte antigens (HLA) typing with high resolution PCR SSP supplementation if they have a potentially appropriate HLA antigen for the study (HLA-A1, A2, B7, B40, Cw7). The high-resolution supplementation will ensure that they are HLA-A*01:01, A*02:01, B*07:02, B*40:01, C*07:02 and thus match the CTL for one or more alleles.

If a patient meets eligibility criteria for the study based on age or comorbid condition(s) and they possess at least 1 HLA allele in common with a cryopreserved CTL product, they will receive viral specific CTLs. Blood for high-resolution typing confirmation will be collected and analyzed. Due to the seriousness of a viral infection, CTL therapy will not be withheld while awaiting the results of central high-resolution confirmatory HLA typing. Patients who do not possess HLA alleles in common with the cryopreserved CTLs will serve as a contemporaneous viral comparison group.

### Example 2: Peptide Preparation

COVID-19 peptides binding to specific HLA alleles are culled from the literature and added to the peptide table. Peptides are added sequentially to the table and given a lab designation that reflects the HLA allele to which it binds plus a two-digit extension after a dash. For example, A*02:01-03 refers to the third peptide on the table which binds to HLA-A*02:01. Peptides used for stimulation of CTL that will be used clinically are purchased from CS Bio or a similar vendor capable of making in vivo grade material.

**Table 1: HLA-A*02:01**

| Peptide Number | Peptide Amino Acid Sequence |
|---|---|
| 01 | ILLNKHIDA* (SEQ ID NO:1) |
| 02 | GMSRIGMEV (SEQ ID NO:2) |
| 03 | ALNTPKDHI (SEQ ID NO:3) |
| 04 | LALLLLDRL (SEQ ID NO:4) |
| 05 | LLLDRLNQL (SEQ ID NO:5) |
| 06 | LQLPQGTTL (SEQ ID NO:6) |
| 07 | FIAGLIAIV (SEQ ID NO:7) |
| 08 | ALNTLVKQL (SEQ ID NO:8) |
| 09 | LITGRLQSL (SEQ ID NO:9) |
| 10 | NLNESLIDL (SEQ ID NO:10) |
| 11 | RLNEVAKNL (SEQ ID NO:11) |
| 12 | VLNDILSRL (SEQ ID NO:12) |
| 13 | VVFLHVTYV (SEQ ID NO:13) |
| 14 | ALSKGVHFV (SEQ ID NO:14) |
| 15 | VLAWLYAAV (SEQ ID NO:15) |
| 16 | KLWAQCVQL (SEQ ID NO:16) |
| 17 | YLQPRTFLL (SEQ ID NO:17) |
| 18 | LLYDANYF (SEQ ID NO:18) |
| 19 | ALWEIQQVV (SEQ ID NO:19) |
| 20 | YLFDESGEFK (SEQ ID NO:20) |
| 21 | FLLN1EMYL (SEQ ID NO:21) |
| 22 | FLLPSLATV (SEQ ID NO:22) |
| 23 | FLAFVVFLL (SEQ ID NO:23) |
| 24 | KLLEQWNLV (SEQ ID NO:24) |
| 25 | SIWNLDYIINL (SEQ ID NO:25) |
| 26 | IFLALITL (SEQ ID NO:26) |
| 27 | FLVFLGIITTV (SEQ ID NO:27) |

**Table 2: HLA-A*01:01**

| Peptide Number | Peptide Amino Acid Sequence |
|---|---|
| 01 | TTDPSFLGRY (SEQ ID NO:28) |
| 02 | LTDEMIAQY (SEQ ID NO:29) |
| 03 | PTDNYITTTY (SEQ ID NO:30) |
| 04 | FTSDYYQLY (SEQ ID NO:31) |
| 05 | ATSRTLSYY (SEQ ID NO:32) |
| 06 | CTDDNALAYY (SEQ ID NO:33) |
| 07 | DTDFVNEFY (SEQ ID NO:34) |
| 08 | NTCDGTTFTY (SEQ ID NO:35) |
| 09 | GTDLEGNFY (SEQ ID NO:36) |
| 10 | RTFKVSIWNLDY (SEQ ID NO:37) |
| 11 | ISEHDYQIGGY (SEQ ID NO:38) |
| 12 | AGDSGFAAY (SEQ ID NO:39) |
| 13 | RQEEVQELY (SEQ ID NO:40) |
| 14 | VDEAGSKSPIQY (SEQ ID NO:41) |
| 15 | SPDDQIGYY (SEQ ID NO:42) |
| 16 | GTGPEAGLPY (SEQ ID NO:43) |
| 17 | LIDLQELGKY (SEQ ID NO:44) |

**Table 3: HLA-B*07:02**

| Peptide Number | Peptide Amino Acid Sequence |
|---|---|
| 01 | SPRWYFYYL (SEQ ID NO:45) |
| 02 | (SEQ ID NO:46) |
| 03 | IPRRNVATL (SEQ ID NO:47) |
| 04 | APHGHVMVEL (SEQ ID NO:48) |
| 05 | TPINLVRDL (SEQ ID NO:49) |
| 06 | APFLYLYAL (SEQ ID NO:50) |
| 07 | KPSFYVYSRV (SEQ ID NO:51) |
| 08 | RPLLESELVI (SEQ ID NO:52) |
| 09 | HPLADNKFAL (SEQ ID NO:53) |
| 10 | EPKLGSLVV (SEQ ID NO:54) |
| 11 | FPRGQGVPI (SEQ ID NO:55) |
| 12 | FPFTIYSLLL (SEQ ID NO:56) |
| 13 | NPANNAAIVL (SEQ ID NO:57) |

**Table 4: HLA-B*40:01**

| Peptide Number | Peptide Amino Acid Sequence |
|---|---|
| 01 | SELVIGAVIL (SEQ ID NO:58) |
| 02 | MEVTPSGTWL (SEQ ID NO:59) |
| 03 | IEYPIIGDEL (SEQ ID NO:60) |
| 04 | AEIVDTVSAL (SEQ ID NO:61) |
| 05 | SEPVLKGVKL (SEQ ID NO:62) |
| 06 | YEGNSPFHPL (SEQ ID NO:63) |

| | |
|---|---|
| 07 | LEYHDVRVVL (SEQ ID NO:64) |
| 08 | NESLIDLQEL (SEQ ID NO:65) |
| 09 | TEAFEKMVSL (SEQ ID NO:66) |
| 10 | TEVPANSTVL (SEQ ID NO:67) |

**Table 5: HLA-C*07:02**

| Peptide Number | Peptide Amino Acid Sequence |
|---|---|
| 01 | NYMPYFFTL (SEQ ID NO:68) |
| 02 | VRFPNITNL (SEQ ID NO:69) |
| 03 | YYQLYSTQL (SEQ ID NO:70) |
| 04 | NRFLYIIKL (SEQ ID NO:71) |
| 05 | IRQEEVQEL (SEQ ID NO:72) |
| 06 | EYHDVRVVL (SEQ ID NO:73) |
| 07 | QRNAPRITF (SEQ ID NO:74) |
| 08 | KKADETQAL (SEQ ID NO:75) |
| 09 | VYDPLQPEL (SEQ ID NO:76) |
| 10 | IYNDKVAGF (SEQ ID NO:77) |

### Example 3: Dendritic Cell Preparation from Monocytes

Dendritic cells are prepared to aid in the generation of specific cytotoxic T lymphocytes (CTLs). We begin typically with 1 x 10⁷ monocytes or multiples thereof. After centrifuging, decant the supernatant and resuspend in complete media (RPMI 1640 supplemented with 10% heat inactivated normal AB serum) along with DNase. Check and record cell count and viability by trypan blue. Transfer to a 6 well plate, then incubate at 37°C, followed by a wash. Add complete media which has been supplemented with GM-CSF and IL-4. The day on which monocytes begin culture in GM-CSF and IL-4 is referred to as Day +1 of the procedure. On the next day (Day +2) add four maturation cytokines IL-6, IL-1 beta, TNF alpha, PGE2. Final concentrations of maturation cytokines vary, but in one case are as follows:

**Table 6: Cytokine Supplementation**

| Cytokine | Final Concentration in Media |
|---|---|
| IL-6 | 1000 IU/mL |
| PGE2 | 1 µg/mL |
| IL-1 beta | 10 ng/mL |
| TNF alpha | 10 ng/mL |

Place the 6-well plate back in the incubator at 37°C. On Day +3, transfer the dendritic cells to a conical tube, centrifuge, decant the supernatant, and resuspend in complete media to which the peptides have been added at a concentration of 2 microgram/mL of each peptide, then incubate. At this point, the dendritic cells are ready for co-culture with lymphocytes. As monocytes mature into dendritic cells, they undergo changes in shape and detach. Approximately half the number of dendritic cells are recovered compared to the starting number of monocytes.

### Example 4: Dendritic cell co-culture with lymphocytes

Dendritic cells prepared in Example 3 are resuspended in complete media with peptides at a cell concentration of 1 x 10⁶ /mL with peptides at a concentration of 2 microgram/mL each. While the dendritic cells are pulsed with peptide, prepare the lymphocytes. Thaw 100 million lymphocytes. Perform cell count and viability tests with trypan blue. The goal is a lymphocyte:monocyte (DC) ratio of 20:1 (100 million:5 million). Lymphocyte viability should be greater than 95%. After centrifugation, resuspend the thawed lymphocytes in complete media with penicillin/streptomycin and transfer to a culture flask, then incubate. After the dendritic cells have been incubated with peptide remove from the incubator, centrifuge again, then resuspend in complete media with penicillin/streptomycin. Add the dendritic cell suspension to a flask which contains the lymphocytes. Incubate the mixture, then add additional complete media with penicillin/streptomycin. After 7 days of co-culture (range 6-8) cells are ready for analysis and re-stimulation.

### Example 5: Stimulation & Enrichment on a Monocyte Monolayer

This procedure is for enrichment of T cells responding to specific peptides on a monocyte monolayer. The T cells can be enriched, activated, and expanded in culture. Beginning with 10 x 10⁶ monocytes, verify cell count and assess viability. Viability should be 92% or higher. Resuspend the monocytes along with DNase in complete media. Incubate, then carefully remove the media. Rinse the monocyte membrane with PBS and subsequently transfer the PBS out of the well. After the PBS is removed, add complete media containing peptides (with each peptide at a 2 microgram/mL concentration) and incubate (during this incubation prepare the lymphocytes). After the incubation the unbound peptides are removed. Peptides are removed as follows: remove the media as described and then wash with PBS.

Harvest the lymphocytes in culture (being careful to gently agitate the flask to get any lymphocytes from the bottom), centrifuge, decant the supernatant and resuspend in complete media and perform a cell count and viability test. Place back in the incubator.

After washing the monocytes, the activated lymphocytes that were transferred into complete media are gently added to the well containing the monocytes and placed in the incubator. Placing the plate in the incubator is the start of T cell selection. Once placed in the incubator, do not disturb the cells in any way during selection.

The selection time to be used is based on the number of lymphocytes undergoing the selection process. If there are less than 90 million lymphocytes, the selection time will be ten minutes. If there are 90 million or more lymphocytes, the selection time will be 7.5 minutes. Once the timer goes off the selection time is complete, and the cells are removed from the incubator.

Remove the media containing the non-adherent cells. After the media is removed, wash with PBS as described above. As before, it is critical to be gentle at this point, as the lymphocytes are adherent to the monocytes and, as the monocytes start to die, the lymphocytes become looser. After the third wash you should notice a white film on the bottom of the plate. This is a good sign (evidence of lymphocytes adhering to the monocytes), and it may be so opaque that one cannot see through the bottom. Exercise caution so as not to let the cells dry out.

Add complete media with penicillin/strep, and place in the incubator. Remove the plate from the incubator and look at the well under the microscope. If you see excessive floating cells remove the media and wash once again with warm PBS. If few or no floating cells observed, incubate overnight. After the fourth wash (if needed), add complete media with pen/strep and incubate overnight.

### T cell expansion

The next day using a transfer pipette, transfer all of the cells remaining in the plate to a tissue culture flask containing complete media with pen/strep containing IL-2. Every 2 days IL-2 should be added to the cultures.

### Example 6: Stimulation with Monocytes in Flasks

This procedure stimulates lymphocytes with monocytes that have been pulsed with peptide in the absence of a T cell enrichment/selection step (such as utilized in Example 5).

Typically, 10 million monocytes are required. Although the ratio of lymphocytes to monocytes may vary somewhat based on available monocytes and the pace of lymphocyte growth after prior stimulations, the ratio will typically be close to 5:1. DNase should be added to the media.

After counting and checking viability, re-suspend the monocytes in complete media containing DNase and to which peptides have been added at a concentration of 2 micrograms/mL for each peptide. The cells should be resuspended in a conical tube. Vortex briefly and place the tube in an incubator. Incubate, centrifuge, then decant the supernatant. Add complete media and vortex to re-suspend. While the monocytes are being pulsed with peptides, the lymphocytes to be stimulated with the monocytes should be counted and viability checked. Re-suspend the lymphocytes in complete media and place in a tissue culture flask. Add the monocytes to the lymphocytes, and then incubate.

Following the above incubation of monocytes and lymphocytes, add complete media with pen/strep containing IL-2. Return the flasks to the incubator. IL-2 should be added to the cultures every two days.

### Example 7: Intracellular Cytokine (ICC) Assay

This procedure is for performing ICC cell preparation prior to staining and performing flow cytometry. Prepare the monocytes before working with the lymphocytes. Thaw monocytes as described in the cell thawing SOP. Count cells and check viability by Trypan Blue. Monocytes must be primed with peptide(s) before the lymphocytes are added (except for the negative control). Always include a negative control in parallel which is monocytes not primed with any peptide.

Re-suspend 0.5 million monocytes in 2 mL of 37 °C complete media in a 5 or 10 mL tube. Add the peptide (for negative control no peptide). The final concentration of each peptide is 2 microgram/mL. If multiple peptides are added to a tube, each should be present at a concentration of 2 microgram/mL. Incubate at 37°C for 90 minutes to "prime the monocytes". This is done at 37°C with the 5 or 10 cc tube lying on its side with the cap loosened and the tube balanced on the top of a 6 well plate. After 90 minutes centrifuge at 10 minutes 482g at room temperature. Decant the supernatant being careful not to decant the monocytes nor to let them dry out after decanting. Add 2 million lymphocytes to each tube (see below for lymphocyte preparation). The ratio of lymphocytes to monocytes is in this example is 4:1 which is appropriate when the frequency of peptide specific lymphocytes is low (below 20%) as would be the case after an initial peptide priming. As the frequency of peptide reactive cells in the lymphocyte population rises with sequential priming, this ratio should decrease. For example, when peptide specific T cells approach purities above 80%, lymphocyte:monocyte ratios should approach 1:1.

### Lymphocyte preparation

Typically, this is started after pulsing the monocytes with peptide and initiating the 90 minute incubation with peptide. Count each "lymphocyte culture". Two million lymphocytes are required for each ICC experiment ("reaction"). Aliquot the appropriate volume for 2 million lymphocytes and centrifuge at 10 minutes at 482g at room temperature. Decant and resuspend in 2 mL of 37°C CM. After decanting the supernatant from the monocytes (above), add these 2 mL to the monocyte tubes. Make sure all the cells are mixed by gentle vortex. Incubate for 2 hours at 37°C in a 5 or 10 cc tube on its side balanced on the top of a 6 well plate with the cap loosened. At the end of 2 hours, add brefeldin A and incubate for 37 degrees for 4 hours.

Stain for ICC. The number of cells reactive with peptide will be very low (1% or less in some cases after initial sensitization) but should rise to much higher levels with repeated stimulation.

### Example 8: Manufacture of 3^{rd} Party viral specific CTLs

Lymphocytes will be exposed to a limited number (up to 20) of peptides known to bind to the HLA restriction element of interest. Lymphocytes are initially stimulated with peptide pulsed dendritic cells and twice more with peptide pulsed monocytes. As part of the first monocyte-peptide re-stimulation, CTLs of interest are enriched due to their preferential adherence to a monocyte-peptide monolayer. The highest frequencies of viral reactive lymphocytes we have seen reported in the literature are less than 2%, (Leen et al., 2006) while our release criteria require at least 20% viral reactive CTLs. Products will be produced by the Jefferson Cell processing laboratory in Philadelphia. Release criteria are as follows: (1) the products must demonstrate that at least 20% of the CTL react to viral peptides in the ICC assay. (Note that reactivity to peptides is always higher in tetramer assays than in ICC assay, and thus the latter is a more rigorous measure). (2) 40% lysis of appropriate target cells at a 20:1 Effector:Target ratio. (3) Flow cytometry must reveal that the cell product contains ≤ 2.5% monocytes, ≤ 2.5% NK cells, ≤ 2.5% naïve T cells. These latter populations were thought to be of concern in triggering GVHD in the BMT studies. While we do not believe they are relevant for the viral population, we include them in our release criteria until more data is available regarding safety.

FIGs. 1A-1C shows the expansion of viral CTLs through the application of the proposed laboratory processes. The graphs illustrate the background level (left panel; FIG. 1A), the detection of a small population of CD8+ T cells after the first week of *in vitro* culture (FIG. 1B) and enrichment to 15% (right panel; FIG. 1C) after selection and further expansion. The x-axis reflects staining with the CD8 marker identifying the cytotoxic subset of T cells. The y-axis reflects interferon-gamma production in response to stimulation with viral peptides capable of binding to HLA-A*02:01. We expect further enrichment will consistently occur with the subsequent stimulation/expansion steps in our process.

### Example 9: Tetramer Assay

Tetramer assays were used to quantitate CTL purity & enrichment. CTL samples were first stained to identify anti-CD8 pMHC tetramers to identify which CTLs have undergone transformation into antigen-specific T-cell populations (viral peptide specific CD8+ T cells). This enables co-staining of antigen-specific T cells and segregation into various phenotypic populations without the distortion oftentimes associated with function-based profiling. CTLs have been generated from all convalescent donors tested. Only 7 of the 20 peptides tested from the COVID-19 genome have been demonstrated to stimulate CTLs. Tetramer assays were used to quantitate CTL purity & enrichment as an alternative to the standard interferon production assay, which was used in other viral models, but has been a poor read out with SARS-CoV-2 (COVID-19) (Habel, J.R. et al. PNAS 2020, 117: 24384-24391.; Keller, M.D. et al. Blood 2020, 136:2905-2917.). The CTLs generated have been greater than 90% CD3+CD8+ and greater than 60% positive in tetramer assays. Tetramer analysis (FIG. 2A) measured CD8+ T cells after a first round of peptide stimulation showing at least ~2.3% of total cells as viral peptide specific CD8+ CTLs. Tetramer analysis (FIG. 2B) further demonstrated that after the final stimulation (third stimulation) greater than 75% of cells in the sample were CD8+ CTLs. Additionally, cell products were greater than or equal to 90% CD8+ and/or CD3+CD8+ after final stimulation.

### Example 10: Cytotoxicity Assay

Functional analysis of virus-specific CTLs was measured as a function of CTL cytotoxicity against viral peptide-pulsed targets. Effector cells were pulsed with 2 µg/mL of viral peptide and incubated at 37°C for a suitable amount of time. After incubation with viral peptides, the media was aspirated and effector cells were rinsed with warm PBS and fresh complete media was replaced. CTLs were titrated into wells seeded with effector cells at 30:1, 10:1, 3:1, and 1:1 ratios (non-viral peptide-pulsed effector cell conditions were kept as controls). Cytotoxicity was measured as a function of release of a radiotracer (51Cr) from cells that had undergone lysis (compared to a control in which all cells are chemically lysed). CTL-mediated cytotoxicity was observed to typically exceed 80% at E:T ratios of 30:1-3:1 (FIG. 3A). Cytotoxicity was approximately 60% at effector to target ratios of 1:1.

### Example 11: Scalability

The approach can be scaled to produce sufficient cells for clinical trials. For example, the proposed phase I-II trial requires about 3.5 x 10⁹ CTLs to support the trial. As shown in Table 7, 2.9 x 10⁹ lymphocytes were isolated from donors and resulted in 9.2 x 10⁹ lymphocytes, at least 76% of which were detectable from tetramer analysis as CD8+, enough material for clinical trials.

**Table 7: Scalability Results**

| Time Point | Lymphocyte Number | CD8+ | Tetramer Positive |
|---|---|---|---|
| Initiation (Prior to Stimulation 1) | 2.9 x 10⁹ viable cells | 39% | Undetectable |
| Completion (Immediately prior to Cryopreservation) | 9.2 x 10⁹ viable cells | 95% | 76% |

### Example 12: Comparative Data

A more focused stimulation, with a limited number of peptides versus using viral infected cells, cells transfected with genes for full length viral proteins, or large peptide libraries was used for comparison. The addition of a selection step using the stimulating viral peptides allowed for greater homogeneity than existing methods and eliminated extraneous lymphocytes that were present in the donor sample.

It was observed that commercially-available virus-specific CTL products are predominantly CD4+ populations, whereas in contrast the method disclosed herein resulted in a statistically significant enrichment for CD8+ T cell populations. CTLs were successfully generated from all convalescent donors to date. Cell products were typically greater than or equal to 90% CD3+CD8+ T cells and greater than 60% positive in tetramer assays (Table 8). Cytotoxicity to peptide pulsed targets exceeded 60% at E:T ratios of 3:1. This approach can be scaled for clinical trials.

As a result, cytotoxicity and lysis responses of cells pulsed with peptides was much stronger than existing methods. We observed nearly four times more killing using less than 1/10th the number of T cells than other CTLs products (nearly 40-fold increase in efficiency). While other commercially-available virus-specific CTL products may elicit cytotoxicity, they often lack identification of which cells in the mixture are important to the treatment. The methods disclosed herein demonstrated that the predominant virus-specific CTLs are entirely the CD8+ subset, cytotoxic, and focused on HLA-class I peptides. Whereas existing products different greatly in the T cell composition, purity, and target identification.

**Table 8. Comparison of product to AlloVir**

| Resultant Product From Our Method | Other Approaches |
|---|---|
| > 90% CD8+ | > 80% CD4+ |
| ➢ 60% Lysis at a 3:1 E:T Ratio | 16% Lysis at a 40:1 E:T Ratio |

### Example 13: Influenza Peptide Preparation

Influenza peptides binding to specific HLA-A2 alleles are culled from the literature and added to the peptide table. Peptides are added sequentially to the table and given a lab designation that reflects the HLA allele to which it binds plus a two-digit extension after a dash. For example, A*02:01-03 refers to the third peptide on the table which binds to HLA-A*02:01. Peptides used for stimulation of CTL that will be used clinically are purchased from CS Bio or a similar vendor capable of making in vivo grade material.

**Table 9. HLA-A2 restricted influenza peptides**

| Peptide Number | Peptide Amino Acid Sequence |
|---|---|
| 1 | GILGFVFTL (SEQ ID NO: 78) |
| 2 | GVLGFVFTL (SEQ ID NO: 79) |
| 3 | GLLGFVFTL (SEQ ID NO: 80) |
| 4 | MSLLTEVETYVLSIVPS (SEQ ID NO: 81) |
| 5 | AGALASCMGLIYNRMGA (SEQ ID NO: 82) |
| 6 | FLKDVMESM (SEQ ID NO: 83) |
| 7 | LEVCFMYSDFHFINEQG (SEQ ID NO: 84) |
| 8 | MDVNPTLLFLKVPAQNA (SEQ ID NO: 85) |
| 9 | AIMDKNIIL (SEQ ID NO: 86) |
| 10 | IMDKNIILKA (SEQ ID NO: 87) |

### Example 22: Manufacture of 3^{rd} Party influenza specific CTLs

Lymphocytes will be exposed to a limited number (up to 20) of peptides known to bind to the HLA restriction element of interest. Lymphocytes are initially stimulated with peptide pulsed dendritic cells and twice more with peptide pulsed monocytes. As part of the first monocyte-peptide re-stimulation, CTLs of interest are enriched due to their preferential adherence to a monocyte-peptide monolayer. The highest frequencies of viral reactive lymphocytes we have seen reported in the literature are less than 2%, (Leen et al., 2006) while our release criteria require at least 20% viral reactive CTLs. Products will be produced by the Jefferson Cell processing laboratory in Philadelphia. Release criteria are as follows: (1) the products must demonstrate that at least 20% of the CTL react to viral peptides in the ICC assay. (Note that reactivity to peptides is always higher in tetramer assays than in ICC assay, and thus the latter is a more rigorous measure). (2) 40% lysis of appropriate target cells at a 20:1 Effector:Target ratio. (3) flow cytometry must reveal that the cell product contains ≤ 2.5% monocytes, ≤ 2.5% NK cells, ≤ 2.5% naïve T cells. These latter populations were thought to be of concern in triggering GVHD in the BMT studies. While we do not believe they are relevant for the viral population, we include them in our release criteria until more data is available regarding safety.

FIG. 4 shows the expansion of influenza CTLs through the application of the proposed laboratory processes. The graph illustrates the detection of a population of CD8+ T cells after a second stimulation. The x-axis reflects staining with the CD8 marker identifying the cytotoxic subset of T cells. The y-axis reflects interferon-gamma production in response to stimulation with influenza peptides capable of binding to HLA-A2.

The entire teachings of all documents cited herein are hereby incorporated herein by reference.
The set of claims as originally filed in the parent application is repeated herein below as clauses in order to preserve all subject matter of the original disclosure in the present divisional application.
Clause 1. A method of treating a viral infection, comprising administering to a human patient in need thereof an effective amount of cells comprising viral peptide specific cytotoxic T lymphocytes (CTLs) that are specifically enriched cells reactive to viral peptides, wherein said CTLs are sensitized against multiple peptides restricted against a single HLA allele by *in vitro* stimulation, wherein at least 20% of said CTLs are reactive to viral peptides, and wherein said cells comprise less than 2.5% of naïve T cells, monocytes, NK cells, or any combination thereof.
Clause 2. The method of clause 1, wherein said human patient is an elderly or immunocompromised patient.
Clause 3. The method of clause 1, wherein said administering is done by intravenous infusion.
Clause 4. The method of clause 3, wherein said infusion is delivered to the patient through a central line or midline.
Clause 5. The method of clause 1, wherein said viral peptide specific CTLs are from a single donor.
Clause 6. The method of any one of clauses 1-5, wherein said viral peptide specific CTLs are sensitized against one or more peptides restricted against an HLA-A1 allele.
Clause 7. The method of clause 6, wherein the one or more peptides are selected from the list consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and a combination thereof.
Clause 8. The method of any one of clauses 1-5, wherein said viral peptide specific CTLs are sensitized against one or more peptides restricted against an HLA-A2 allele.
Clause 9. The method of clause 8, wherein the one or more peptides are selected from the list consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and a combination thereof.
Clause 10. The method of any one of clauses 1-5, wherein said viral peptide specific CTLs are sensitized against one or more peptides restricted against an HLA-B7 allele.
Clause 11. The method of clause 10, wherein the one or more peptides are selected from the list consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, and a combination thereof.
Clause 12. The method of any one of clauses 1-5, wherein said viral peptide specific CTLs are sensitized against one or more peptides restricted against an HLA-B40 allele.
Clause 13. The method of clause 12, wherein the one or more peptides selected from the list consisting of SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and a combination thereof.
Clause 14. The method of any one of clauses 1-5, wherein said viral peptide specific CTLs are sensitized against one or more peptides restricted against an HLA-Cw7 allele.
Clause 15. The method of clause 14, wherein the one or more peptides are selected from the list consisting of SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, and a combination thereof.
Clause 16. The method of any one of clauses 1-5, wherein said viral peptide specific CTLs are sensitized against a combination of viral peptides binding to any one or combination of HLA-A1, A2, B7, B40, Cw7 alleles.
Clause 17. The method of any one of clauses 1-16, wherein the viral peptide is from a severe acute respiratory syndrome (SARS) virus.
Clause 18. The method of clause 17, wherein the viral peptide is from a SARS-coronavirus 2 (COVID-19) virus.
Clause 19. The method of clause 8, wherein the one or more peptides are selected from the list consisting of SEQ ID NOs 78-87, and a combination thereof.
Clause 20. A method of preparing viral peptide specific cytotoxic T cells (CTLs) that are specifically enriched cells reactive to viral peptides comprising:
   a. a first stimulation step, whereby a subset of monocytes are treated to induce maturation into dendritic cells, the dendritic cells are pulsed with one or more virus specific peptides and co-cultured with lymphocytes for at least six days;
   b. a second stimulation step, whereby monocytes are used to present the viral specific peptides, stimulated lymphocytes are cultured for at least seven days, peptide specific CTLs are selected due to preferential adherence of T cells recognizing the pulse peptides to an adherent monocyte layer; and
   c. a third stimulation step, whereby a subset of monocytes are pulsed with multiple viral specific peptides; restricted against a single HLA allele; thereby producing viral peptide specific CTLs, wherein at least 20% of the CTLs are reactive to viral peptides.
Clause 21. The method of clause 20, wherein the viral reactive CTLs are allogeneic mononuclear leukocytes collected from a single donor.
Clause 22. The method of clause 20 or 21, wherein inducing maturation into dendritic cells comprises a first treatment of the monocytes with GM-CSF, IL-4, or a combination of the two, for at least about 24 hours, followed by a second treatment of the monocytes with TNF-alpha, IL-1 beta, IL-6, prostaglandin E2, or any combination thereof for at least about 24 hours after the first treatment.
Clause 23. The method of any one of clauses 20-22, wherein pulsing the dendritic cells with viral peptides comprises incubating the dendritic cells with at least about 2 µg/mL for each viral peptide.
Clause 24. The method of any one of clauses 20-23, wherein stimulated lymphocytes in the second stimulation step are further selected for by treating the co-culture with human interleukin-1 (IL-1).
Clause 25. The method of any one of clauses 20-24, wherein said viral peptide specific CTLs are sensitized against one or more peptides restricted against an HLA-A1 allele.
Clause 26. The method of clause 25, wherein the one or more peptides are selected from the list consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and a combination thereof.
Clause 27. The method of any one of clauses 20-24, wherein said viral peptide specific CTLs are sensitized against one or more peptides restricted against an HLA-A2 allele.
Clause 28. The method of clause 27, wherein the one or more peptides are selected from the list consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and a combination thereof.
Clause 29. The method of any one of clauses 20-24, wherein said viral peptide specific CTLs are sensitized against one or more peptides restricted against an HLA-B7 allele.
Clause 30. The method of clause 29, wherein the one or more peptides are selected from the list consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, and a combination thereof.
Clause 31. The method of any one of clauses 20-24, wherein said viral peptide specific CTLs are sensitized against one or more peptides restricted against an HLA-B40 allele.
Clause 32. The method of clause 31, wherein the one or more peptides are selected from the list consisting of SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and a combination thereof.
Clause 33. The method of any one of clauses 20-24, wherein said viral peptide specific CTLs are sensitized against one or more peptides restricted against an HLA-Cw7 allele.
Clause 34. The method of clause 33, wherein the one or more peptides are selected from the list consisting of SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, and a combination thereof.
Clause 35. The method of any one of clauses 20-24, wherein the viral peptide specific CTLs are sensitized against a combination of viral peptides binding to any one or more of a combination of HLA-A1, A2, B7, B40, Cw7 alleles.
Clause 36. The method of any one of clauses 20-35, wherein the viral peptide is from a severe acute respiratory syndrome (SARS) virus.
Clause 37. The method of clause 36, wherein the viral peptide is from a SARS-coronavirus 2 (COVID-19) virus.
Clause 38. The method of clause 27, wherein the one or more peptides are selected from the list consisting of SEQ ID NOS 78-87, and a combination thereof.
Clause 39. A pharmaceutical composition comprising cells comprising viral peptide specific cytotoxic T lymphocytes (CTLs) that are specifically enriched cells reactive to viral peptides, wherein said CTLs are sensitized against multiple peptides restricted against a single HLA allele by *in vitro* stimulation, wherein at least 20% of said CTLs are reactive to viral peptides, and wherein said cells comprise less than 2.5% of naïve T cells, monocytes, NK cells, or any combination thereof.
Clause 40. The pharmaceutical composition of clause 39, wherein said CTLs are from multiple donors.
Clause 41. The pharmaceutical composition of clause 39 or 40, wherein the CTLs have been sensitized against one or more viral peptides binding to specific HLA-A1 alleles selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO: 19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and a combination thereof.
Clause 42. The pharmaceutical composition of clause 39 or 40, wherein the CTLs have been sensitized against one or more viral peptides binding to specific HLA-A2 alleles selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and a combination thereof.
Clause 43. The pharmaceutical composition of clause 39 or 40, wherein the CTLs have been sensitized against one or more viral peptides binding to specific HLA-B7 alleles selected from the group consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, and a combination thereof.
Clause 44. The pharmaceutical composition of clause 39 or 40, wherein the CTLs have been sensitized against one or more viral peptides binding to specific HLA-B40 alleles selected from the group consisting of SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and a combination thereof.
Clause 45. The pharmaceutical composition of clause 39 or 40, wherein the CTLs have been sensitized against one or more viral peptides binding to specific HLA-Cw7 alleles selected from the group consisting of SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, and a combination thereof.
Clause 46. The pharmaceutical composition of clause 39 or 40, wherein the CTLs have been sensitized against one or more viral peptides binding to any one or a combination of HLA-A1, A2, B7, B40, or Cw7 alleles.
Clause 47. The pharmaceutical composition of clause 39 or 40, wherein the pharmaceutical composition comprises cryopreserved CTLs in DMSO, RPMl-1640, albumin, or a combination thereof.
Clause 48. The pharmaceutical composition of clause 39 or 40, wherein the pharmaceutical composition comprises one or more additional anti-viral agents.
Clause 49. The pharmaceutical composition of clause 39 or 40, wherein the pharmaceutical composition is in a form that is suitable for intravenous administration.
Clause 50. The pharmaceutical composition of clause 39 or 40, wherein the viral peptide specific CTLs are specific for a virus selected from the group consisting of SARS-CoV-2 (COVID-19), influenza, parainfluenza, respiratory syncytial virus (RSV), metapneumovirus, Hepatitis B virus (HBV), Epstein-Barr virus (EBV), cytomegalovirus (CMV), BK virus (BKV), John Cunningham virus (JCV), human herpesvirus (HHV), and adenovirus.
Clause 51. The pharmaceutical composition of clause 39 or 40, wherein the viral peptide is from a severe acute respiratory syndrome (SARS) virus.
Clause 52. The pharmaceutical composition of clause 51, wherein the viral peptide is from a SARS-coronavirus 2 (COVID-19) virus.
Clause 53. The pharmaceutical composition of clause 39 or 40, wherein the CTLs have been sensitized against one or more viral peptides binding to specific HLA-A2 alleles selected from the group consisting of SEQ ID NOS 78-87, and a combination thereof.

## Claims

1. Cells comprising viral peptide specific cytotoxic T lymphocytes (CTLs) that are specifically enriched cells reactive to viral peptides for use in a method of treating a viral infection, wherein an effective amount of said cells is to be administered to a human patient in need thereof, wherein said CTLs are sensitized against multiple peptides restricted against a single HLA allele by *in vitro* stimulation, wherein at least 20% of said CTLs are reactive to viral peptides, and wherein said cells comprise less than 2.5% of naïve T cells, monocytes, NK cells, or any combination thereof.

2. The cells comprising viral peptide specific cytotoxic T lymphocytes (CTLs) that are specifically enriched cells reactive to viral peptides for use according to claim 1, wherein said human patient is an elderly or immunocompromised patient, more particularly an adult over 60 years; a patient with serious chronic medical conditions such as heart disease, diabetes, and lung disease; a cancer patient; a patient with pulmonary disease such as asthma, airway hyperresponsiveness, allergic rhinitis, bronchiectasis, chronic bronchitis, emphysema, chronic obstructive pulmonary disease, cystic fibrosis, and early life wheezing; or belongs to any such patient population particularly susceptible to viral and other respiratory infections.

3. The cells comprising viral peptide specific cytotoxic T lymphocytes (CTLs) that are specifically enriched cells reactive to viral peptides for use according to claim 1, wherein said administering is done by intravenous infusion, optionally wherein said infusion is delivered to the patient through a central line or midline.

4. The cells comprising viral peptide specific cytotoxic T lymphocytes (CTLs) that are specifically enriched cells reactive to viral peptides for use according to claim 1, wherein said viral peptide specific CTLs are from a single donor.

5. The cells comprising viral peptide specific cytotoxic T lymphocytes (CTLs) that are specifically enriched cells reactive to viral peptides for use according to any one of claims 1-4, wherein said viral peptide specific CTLs are sensitized against one or more peptides restricted against an HLA-A1 allele, optionally wherein the one or more peptides are selected from the list consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and a combination thereof, or
wherein said viral peptide specific CTLs are sensitized against one or more peptides restricted against an HLA-A2 allele, optionally wherein the one or more peptides are selected from the list consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and a combination thereof, or optionally wherein the one or more peptides are selected from the list consisting of SEQ ID NOs 78-87, and a combination thereof.

6. The cells comprising viral peptide specific cytotoxic T lymphocytes (CTLs) that are specifically enriched cells reactive to viral peptides for use according to any one of claims 1-4, wherein said viral peptide specific CTLs are sensitized against one or more peptides restricted against an HLA-B7 allele, optionally wherein the one or more peptides are selected from the list consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, and a combination thereof, or
wherein said viral peptide specific CTLs are sensitized against one or more peptides restricted against an HLA-B40 allele, optionally wherein the one or more peptides selected from the list consisting of SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and a combination thereof.

7. The cells comprising viral peptide specific cytotoxic T lymphocytes (CTLs) that are specifically enriched cells reactive to viral peptides for use according to any one of claims 1-4, wherein said viral peptide specific CTLs are sensitized against one or more peptides restricted against an HLA-Cw7 allele, optionally wherein the one or more peptides are selected from the list consisting of SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, and a combination thereof, or
wherein said viral peptide specific CTLs are sensitized against a combination of viral peptides binding to any one or combination of HLA-A1, A2, B7, B40, Cw7 alleles.

8. The cells comprising viral peptide specific cytotoxic T lymphocytes (CTLs) that are specifically enriched cells reactive to viral peptides for use according to any one of claims 1-7, wherein the viral peptide is from a severe acute respiratory syndrome (SARS) virus, optionally wherein the viral peptide is from a SARS-coronavirus 2 (COVID-19) virus.

9. A method of preparing viral peptide specific cytotoxic T cells (CTLs) that are specifically enriched cells reactive to viral peptides comprising:
a. a first stimulation step, whereby a subset of monocytes are treated to induce maturation into dendritic cells, the dendritic cells are pulsed with one or more virus specific peptides and co-cultured with lymphocytes for at least six days;
b. a second stimulation step, whereby monocytes are used to present the viral specific peptides, stimulated lymphocytes are cultured for at least seven days, peptide specific CTLs are selected due to preferential adherence of T cells recognizing the pulse peptides to an adherent monocyte layer; and
c. a third stimulation step, whereby a subset of monocytes are pulsed with multiple viral specific peptides; restricted against a single HLA allele; thereby producing viral peptide specific CTLs, wherein at least 20% of the CTLs are reactive to viral peptides, optionally wherein the viral reactive CTLs are allogeneic mononuclear leukocytes collected from a single donor, optionally wherein inducing maturation into dendritic cells comprises a first treatment of the monocytes with GM-CSF, IL-4, or a combination of the two, for at least about 24 hours, followed by a second treatment of the monocytes with TNF-alpha, IL-1 beta, IL-6, prostaglandin E2, or any combination thereof for at least about 24 hours after the first treatment.

10. The method of claim 9, wherein pulsing the dendritic cells with viral peptides comprises incubating the dendritic cells with at least about 2 µg/mL for each viral peptide, and/or
wherein stimulated lymphocytes in the second stimulation step are further selected for by treating the co-culture with human interleukin-1 (IL-1) and optionally the final concentration of IL-1 in media is 10 ng/mL.

11. The method of claim 9 or claim 10, wherein said viral peptide specific CTLs are sensitized against one or more peptides restricted against an HLA-A1 allele, optionally wherein the one or more peptides are selected from the list consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and a combination thereof, or
wherein said viral peptide specific CTLs are sensitized against one or more peptides restricted against an HLA-A2 allele, optionally wherein the one or more peptides are selected from the list consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and a combination thereof, or optionally wherein the one or more peptides are selected from the list consisting of SEQ ID NOS 78-87, and a combination thereof, or
wherein said viral peptide specific CTLs are sensitized against one or more peptides restricted against an HLA-B7 allele, optionally wherein the one or more peptides are selected from the list consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, and a combination thereof, or
wherein said viral peptide specific CTLs are sensitized against one or more peptides restricted against an HLA-B40 allele, optionally wherein the one or more peptides are selected from the list consisting of SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and a combination thereof.

12. The method of claim 9 or claim 10, wherein said viral peptide specific CTLs are sensitized against one or more peptides restricted against an HLA-Cw7 allele, optionally wherein the one or more peptides are selected from the list consisting of SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, and a combination thereof, or
wherein the viral peptide specific CTLs are sensitized against a combination of viral peptides binding to any one or more of a combination of HLA-A1, A2, B7, B40, Cw7 alleles, and/or
wherein the viral peptide is from a severe acute respiratory syndrome (SARS) virus, optionally wherein the viral peptide is from a SARS-coronavirus 2 (COVID-19) virus.

13. A pharmaceutical composition comprising cells comprising viral peptide specific cytotoxic T lymphocytes (CTLs) that are specifically enriched cells reactive to viral peptides, wherein said CTLs are sensitized against multiple peptides restricted against a single HLA allele by in vitro stimulation, wherein at least 20% of said CTLs are reactive to viral peptides, and wherein said cells comprise less than 2.5% of naïve T cells, monocytes, NK cells, or any combination thereof, optionally wherein said CTLs are from multiple donors.

14. The pharmaceutical composition of claim 13, wherein the CTLs have been sensitized against one or more viral peptides binding to specific HLA-A1 alleles selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO: 19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and a combination thereof, or
wherein the CTLs have been sensitized against one or more viral peptides binding to specific HLA-A2 alleles selected from the group consisting of SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, and a combination thereof, or
wherein the CTLs have been sensitized against one or more viral peptides binding to specific HLA-B7 alleles selected from the group consisting of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, and a combination thereof, or
wherein the CTLs have been sensitized against one or more viral peptides binding to specific HLA-B40 alleles selected from the group consisting of SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and a combination thereof, or
wherein the CTLs have been sensitized against one or more viral peptides binding to specific HLA-Cw7 alleles selected from the group consisting of SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, and a combination thereof, or
wherein the CTLs have been sensitized against one or more viral peptides binding to any one or a combination of HLA-A1, A2, B7, B40, or Cw7 alleles, or
wherein the pharmaceutical composition comprises cryopreserved CTLs in DMSO, RPMl-1640, albumin, or a combination thereof, or
wherein the pharmaceutical composition comprises one or more additional anti-viral agents, or
wherein the pharmaceutical composition is in a form that is suitable for intravenous administration, or
wherein the viral peptide specific CTLs are specific for a virus selected from the group consisting of SARS-CoV-2 (COVID-19), influenza, parainfluenza, respiratory syncytial virus (RSV), metapneumovirus, Hepatitis B virus (HBV), Epstein-Barr virus (EBV), cytomegalovirus (CMV), BK virus (BKV), John Cunningham virus (JCV), human herpesvirus (HHV), and adenovirus, or
wherein the viral peptide is from a severe acute respiratory syndrome (SARS) virus, optionally wherein the viral peptide is from a SARS-coronavirus 2 (COVID-19) virus.

15. The pharmaceutical composition of claim 13, wherein the CTLs have been sensitized against one or more viral peptides binding to specific HLA-A2 alleles selected from the group consisting of SEQ ID NOS 78-87, and a combination thereof.
